Europäisches Patentamt

European Patent Office

Office européen des brevets

(11) Veröffentlichungsnummer: **0 280 783**
**A1**

## (12) EUROPÄISCHE PATENTANMELDUNG

(21) Anmeldenummer: 87119105.2

(22) Anmeldetag: 23.12.87

(51) Int. Cl.4 **A61M 1/00 , A61F 9/00**

(30) Priorität: 16.01.87 DE 8700744 U

(43) Veröffentlichungstag der Anmeldung:
07.09.88 Patentblatt 88/36

(84) Benannte Vertragsstaaten:
CH DE FR GB IT LI NL

(71) Anmelder: **Erbe Elektromedizin GmbH.**
**Waldhörnlestrasse 17**
**D-7400 Tübingen(DE)**

(72) Erfinder: **van Gerven, Johannes Theodorus**
**Maria**
**Eugenstrasse 10**
**D-7403 Ammerbuch 1(DE)**

(74) Vertreter: **Endlich, Fritz, Dipl.-Phys.**
**Postfach 1326 Blumenstrasse 8**
**D-8034 Germering(DE)**

(54) **Handgriff mit einer Doppelkanüle.**

(57) Es wird ein Handgriff mit einer Doppelkanüle zum Absaugen von Linsentrümmern bei der Durchführung von Linsenstar-Operationen beschrieben, wobei die Doppelkanüle aus zwei koaxialen Röhrchen (1,2) besteht. Das innere Röhrchen (1) hat eine geschlossene Spitze und eine seitlich angeordnete Saugöffnung (3). Das vordere Ende des äußeren Röhrchens (2) ist in einem Abstand von der Spitze mit dem inneren Röhrchen (1) verbunden. Es sind zwei seitlich angeordnete Spülöffnungen (4) vorgesehen, die in Umfangsrichtung der Doppelkanüle relativ zu der Saugöffnung (3) zumindest angenähert um 45° versetzt zu der Saugöffnung (3) angeordnet sind. Auf der den beiden Spülöffnungen (4) gegenüberliegenden Seite des äußeren Röhrchens (2) ist ein als Irishäkchen dienender Anschlag (5) ausgebildet. Das vorragende Ende des inneren Röhrchens (1) kann geradlinig oder abgewinkelt oder abgebogen ausgebildet sein. Der Anschlag (5) kann halbkugelförmig ausgebildet werden.

FIG. 1

## Handgriff mit einer Doppelkanüle

Die Neuerung betrifft einen Handgriff mit einer Doppelkanüle zum Absaugen von Linsentrümmern bei der Durchführung von Linsenstar-Operationen.

Es ist bereits eine Apparatur mit einem derartigen Handgriff mit einer Doppelkanüle zum Absaugen von Linsentrümmern bekannt (DE-GM 84 37 836), wobei die Doppelkanüle aus zwei koaxialen Röhrchen besteht, wovon das innere Röhrchen eine geschlossene Spitze und eine seitlich angeordnete Saugöffnung aufweist. Das vordere Ende des äußeren Röhrchens ist in einem Abstand von der Spitze mit dem inneren Röhrchen verbunden und weist zwei seitlich angeordnete Spülöffnungen auf, die in Umfangsrichtung der Doppelkanüle relativ zu der Saugöffnungen auf, die in Umfangsrichtung der Doppelkanüle relativ zu der Saugöffnung zumindest angenähert um 45° versetzt angeordnet sind.

Bekanntliche liegt die Iris nach Art einer Blende vor der Linse. Ihre als Pupille bezeichnete Öffnung kann normalerweise durch verengend oder erweiternd wirkende Muskeln je nach Bedarf verändert werden. Während des Absaugens von Linsenresten bei der Durchführung einer Katarakt-Operation besteht die Schwierigkeit, daß die durch die Iris begrenzte Öffnung anfänglich größer ist, aber nach einer gewissen Zeit wieder kleiner wird, und daß mit Medikamenten eine Vergrößerung bewirkt werden kann. Dadurch können Schwierigkeiten verursacht werden, die den Erfolg der Operation beeinträchtigen können.

Es ist deshalb Aufgabe der Neuerung, einen mit einer Doppelkanüle versehenen Handgriff zum Absaugen von Linsentrümmern bei der Durchführung von Linsenstar-Operationen derart zu verbessern, daß eine zweckmäßigere Handhabung möglich ist, durch die verbesserte Operationserfolge im Hinblick auf die unvermeidbare Verkleinerung der Pupille erzielbar sind.

Diese Aufgabe wird bei einem Handgriff der eingangs genannten Art durch die Neuerung dadurch gelöst, daß auf der den beiden Spülöffnungen gegenüberliegenden Seite des äußeren Röhrchens ein als Irishäkchen dienender Anschlag ausgebildet ist. Vorzugsweise verläuft die Achse der beiden koaxialen Röhrchen bogenförmig, um ein einfacheres Eindringen in Augenkammerwinkel zu ermöglichen und dadurch die Wirkung durch verbesserte Handhabungsmöglichkeiten weiter zu begünstigen.

Anhand der Zeichnung soll die Neuerung beispielsweise näher erläutert werden. Es zeigen:

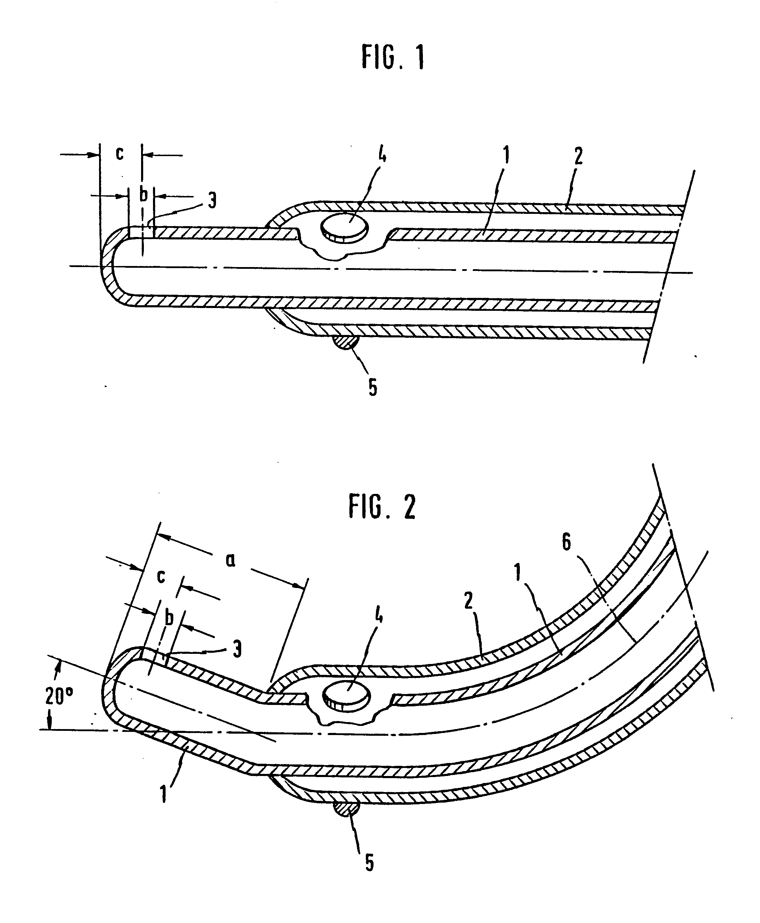

Fig. 1 und 2 Schnittansichten von zwei Ausführungsbeispielen gemäß der Neuerung.

Bei dem in Fig. 1 dargestellten Ausführungsbeispiel ist eine geradlinige Anordnung der beiden koaxial zueinander angeordneten Röhrchen 1 und 2 vorgesehen. Das innere Röhrchen 1 weist eine geschlossene Spitze auf und eine seitlich angeordnete Saugöffnung 3. Der Abstand c in Fig. 1 kann beispielsweise 0,5mm und der durchmesser b kann 0,3 mm betragen. Im allgemeinen beträgt der Abstand c zwischen 0,5 und 0,7 mm und der Durchmesser b liegt zwischen 0,3 und 0,7 mm. Das äußere Röhrchen 2 ist angrenzend an sein vorderes Ende mit zwei Spülöffnungen 4 versehen, die vorzugsweise um 45° versetzt zu der Saugöffnung 3 angeordnet sind, und von denen nur eine in Fig. 1 sichtbar ist. Der Durchmesser der beiden Spülöffnungen 4 beträgt bei dem dargestellten Ausführungsbeispiel 0,6 mm, und die Spitze des inneren Röhrchens 1 kann einen Abstand von dem vorderen Ende des äußeren Röhrchens zwischen 1,3 und 1,5 mm aufweisen.

Wenn die beiden Spülöffnungen 4 angenähert um 45° versetzt zu der Saugöffnung 3 sind, kann eine intensive Spülung in der Vorderkammer des Auges erzielt werden, weil beide Spülströme auf die Innenwand der hinteren Linsenkapsel gerichtet werden können, so daß entlang des verhältnismäßig kurzen Wegs zwischen den beiden Spülöffnungen 4 und der dazwischen liegenden Saugöffnung 3 beidseitig eine zu der Saugöffnung gerichtete Wirbelströmung er zielt werden kann, die eine intensive Spülung und damit eine bessere Entfernung der Linsentrümmer ermöglicht.

Gemäß der Neuerung ist auf der den beiden Spülöffnungen 4 gegenüberliegenden Seite des äußeren Röhrchens 2 ein als Irishäkchen dienender Anschlag 5 vorgesehen, so daß die Iris auseinandergezogen werden kann, wenn sie bei einer Operation nach einer gewissen Zeit wieder kleiner wird.

Ein bevorzugtes Ausführungsbeispiel ist in Fig. 2 dargestellt, wobei das vorragende Ende des inneren Röhrchens um etwa 20° abgewinkelt oder abgebogen ist. Ferner verläuft die Achse 6 der beiden koaxialen Röhrchen 1,2 bogenförmig, so daß ein besseres Eindringen in Augenkammerwinkel erzielbar ist, da dann die Nase des Patienten dabei nicht stört.

## Ansprüche

1. Handgriff mit einer Doppelkanüle zum Absaugen von Linsentrümmern bei der Durchführung von Linsenstar-Operationen, wobei die Doppelkanüle aus zwei koaxialen Röhrchen besteht, wovon das innere Röhrchen eine geschlossene Spitze und eine seitlich angeordnete Saugöffnung aufweist, und das vordere Ende des äußeren Röhrchens in einem Abstand von der Spitze mit dem inneren Röhrchen verbunden ist und zwei seitlich angeordnete Spülöffnungen aufweist, die in Umfangsrichtung der Doppelkanüle relativ zu der Saugöffnung zumindest angenähert um 45° versetzt zu der Saugöffnung angeordnet sind, **dadurch gekennzeichnet,** daß auf der den beiden Spülöffnungen (4) gegenüberliegenden Seite des äußeren Röhrchens (2) ein als Irishäkchen dienender Anschlag (5) ausgebildet ist.

2. Handgriff nach Anspruch 1, **dadurch gekennzeichnet,** daß das vorragende Ende des inneren Röhrchens (1) geradlinig ausgebildet ist.

3. Handgriff nach Anspruch 1, **dadurch gekennzeichnet,** daß das vorragende Ende des inneren Röhrchens (1) abgewinkelt oder abgebogen ausgebildet ist.

4. Handgriff nach einem der vorhergenden Ansprüche, **dadurch gekennzeichnet,** daß die Achse (6) der beiden koaxialen Röhrchen (1,2) bogenförmig verläuft.

5. Handgriff nach einem der vorhergenden Ansprüche, **dadurch gekennzeichnet,** daß der Anschlag(5) halbkugelförmig ausgebildet ist.

# FIG. 1

# FIG. 2

| EINSCHLÄGIGE DOKUMENTE | | EP 87119105.5 | |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| D,A | <u>DE - U1 - 8 437 836</u> (ERBE ELEKTRO-MED.) <br> * Fig. 1-4; Schutzansprüche 1-3 * <br><br> -- | 1-3 | A 61 M 1/00 <br> A 61 F 9/00 |
| A | <u>US - A - 4 634 423</u> (P.F.BAILEY JR.) <br> * Fig. 2,8; Spalte 4, Zeilen 31,32; Spalte 6, Zeilen 15-22 * <br><br> ---- | 1 | |

| | RECHERCHIERTE SACHGEBIETE (Int. Cl.4) |
|---|---|
| | A 61 B 17/00 <br> A 61 F 9/00 <br> A 61 M 1/00 <br> A 61 M 7/00 |

Der vorliegende Recherchenbericht wurde für alle Patentansprüche erstellt.

| Recherchenort | Abschlußdatum der Recherche | Prüfer |
|---|---|---|
| WIEN | 13-06-1988 | LUDWIG |